# EUROPEAN PATENT SPECIFICATION

(11) **EP 1 200 439 B1**
(45) Date of publication and mention of the grant of the patent: **25.06.2003**
(21) Application number: 00949428.7
(22) Date of filing: 31.07.2000
(51) Int. Cl.: C07D 487/04, A61K 31/4985, A61P 35/00

(54) **1,2-DIHYDRO-1-OXO-PARAZINO(1,2-A) INDOLE DERIVATIVES**
1,2-DIHYDRO-1-OXO-PYRAZINO[1,2-]INDOLEDERIVATE
DERIVES 1,2-DIHYDRO-1-OXO-PYRAZINO 1,2-A]INDOLE

(30) Priority: 03.08.1999 IT MI991741
(43) Date of publication of application: 02.05.2002
(73) Proprietor: Novuspharma S.p.A., 20131 Milano (IT)
(72) Inventor: MENTA, Ernesto, I-20052 Monza (IT); NICOTRA, Francesco, I-20052 Monza (IT); PESCALLI, Nicoletta, I-20052 Monza (IT); SPINELLI, Silvano, I-20052 Monza (IT)
(74) Representative: Minoja, Fabrizio, Dr.
(86) International application number: EP0007418
(87) International publication number: WO01009136

(56) References cited:
- WO-A-94/22821
- VITI, GIOVANNI ET AL: "Synthesis of tetracyclic arylpyridodiazepines as potential anti-HIV-1 agents" HETEROCYCLES (1995), 41(4), 753-63 , XP002156349

## Description

The present invention relates to 1,2-dihydro-1-oxo-pyrazino[1,2-a]indole derivatives and to the pharmaceutical compositions containing them.

### PRIOR ART

Indole derivatives endowed with inhibitory activity on 5-α-reductase and useful for the treatment of 5-α-reductase-mediated diseases, among them prostatic cancer, are disclosed in WO 94/22821.

Pyrazino[1,2-a]indol-1-one derivatives such as 3-hydroxymethyl-2-methyl-pyrazino[1,2-a]indol-1(2H) -one, 1,2-dihydro-2-methyl-3-carboxymethylpirazino[1,2-a]indol-1(2H)-one and 1,2-dihydro-3-carboxymethylpyrazino[1,2-a]indol-1(2H)-one are described in Tetrahedron, 1994, (5), 4887-96. None of these compounds has been disclosed to have antitumor activity.

It has now been found that some 1,2-dihydro-1-oxo-pyrazino[1,2-a]indoles have antitumor activity, particularly against colon and lung tumors.

### DISCLOSURE OF THE INVENTION

The present invention relates to compounds of formula (I), 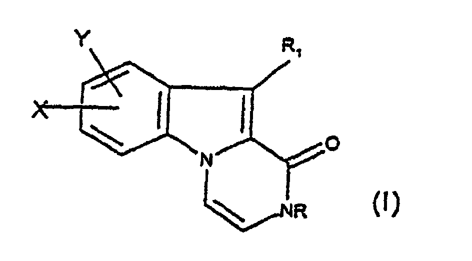 wherein:
- R: is hydrogen; C₁-C₆ alkyl optionally substituted with one or more primary, secondary or tertiary amino groups, hydroxy or carboxy groups; C₅-C₆ cycloalkyl; aralkyl ; aryl; heteroaryl; heteroaralkyl;
- R₁: is hydrogen; C₁-C₆ alkyl optionally substituted with one or more primary, secondary or tertiary amino groups, hydroxy or carboxy groups; C₅-C₆ cycloalkyl; aralkyl; halogen;
- Y: is hydrogen or a -O-R₂ group;
- R₂: is hydrogen; C₁-C₆ alkyl optionally substituted with one or more primary, secondary or tertiary amino groups, hydroxy or carboxy groups; C₅-C₆ cycloalkyl; aralkyl; aryl; heteroaryl; heteroarylalkyl; C₁-C₆ acyl;

X is one or more groups independently selected from hydrogen, C₁-C₆ alkyl, hydroxy, C₁-C₄ alkoxy, C₁-C₃ haloalkoxy, amino, C₁-C₃ alkylamino, C₁-C₃-acylamino, C₁-C₃- alkylsulfonylamino, halogen, nitro, cyano, carboxy, C₁-C₃ alkoxycarbonyl, sulfonyl, C₁-C₃-alkyl-sulfonyl, aminosulfonyl, C₁-C₃- alkylaminosulfonyl, trifluoromethyl.

C₁-C₆ Alkyl is preferably methyl, ethyl, n-propyl, isopropyl, isobutyl, in particular methyl.

Substituted alkyl is preferably 2-hydroxyethyl, 2-aminoethyl, 2-dimethylaminoethyl, carboxymethyl.

C₅-C₆ Cycloalkyl is cyclohexyl or cyclopentyl.

Aralkyl is benzyl or phenethyl.

Aryl is preferably phenyl, naphthyl or phenyl substituted with one or two substituents selected from C₁-C₆ alkyl, hydroxy, C₁-C₄ alkoxy, C₁-C₃ haloalkoxy, amino, C₁-C₃ alkylamino, C₁-C₃ acylamino, C₁-C₃-alkylsulfonylamino, halogen, nitro, cyano, carboxy, C₁-C₃ alkoxycarbonyl, sulfonyl, C₁-C₃-alkylsulfonyl, aminosulfonyll, C₁-C₃-alkylaminosulfonyl, trifluoromethyl.

Heteroaryl is furyl, thienyl, imidazolyl, benzothienyl, benzofuryl, pyridyl, indolyl, pyrimidyl, thiazolyl.

Heteroarylalkyl is pyridylmethyl, thienylmethyl, furylmethyl, isoquinolylmethyl.

C₁-C₄ Alkoxy is preferably methoxy or ethoxy.

C₁-C₃ Haloalkoxy is preferably difluoromethoxy, trichloromethoxy, trifluoromethoxy.

Halogen is preferably chlorine.

C₁-C₃ alkylamino is preferably methylamino, C₁-C₃ acylamino is preferably acetylamino, C₁-C₃-alkylsulfonylamino is preferably methylsulfonylamino, C₁-C₃-alkylsulfonyl is preferably methylsulfonyl, C₁-C₃-alkylaminosulfonyl is preferably methylaminosulfonyl and dimethylaminosulfonyl.

Examples of preferred compounds of formula (I) are those in which R is C₁-C₆ alkyl, R₁ is hydrogen or methyl, X is hydrogen, Y is OR₂ and R₂ is C₁-C₆ alkyl, aralkyl or aryl. For Y=O-R₂, R₂ is preferably at the 8 position of the 1,2-dihydro-1-oxo-pyrazino-[1,2-a]indole heterocyclic system, as represented in the following formula (Ia)

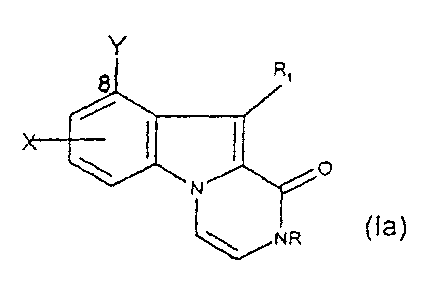

The compounds of formula (I) can be prepared according to the process illustrated in the following scheme:

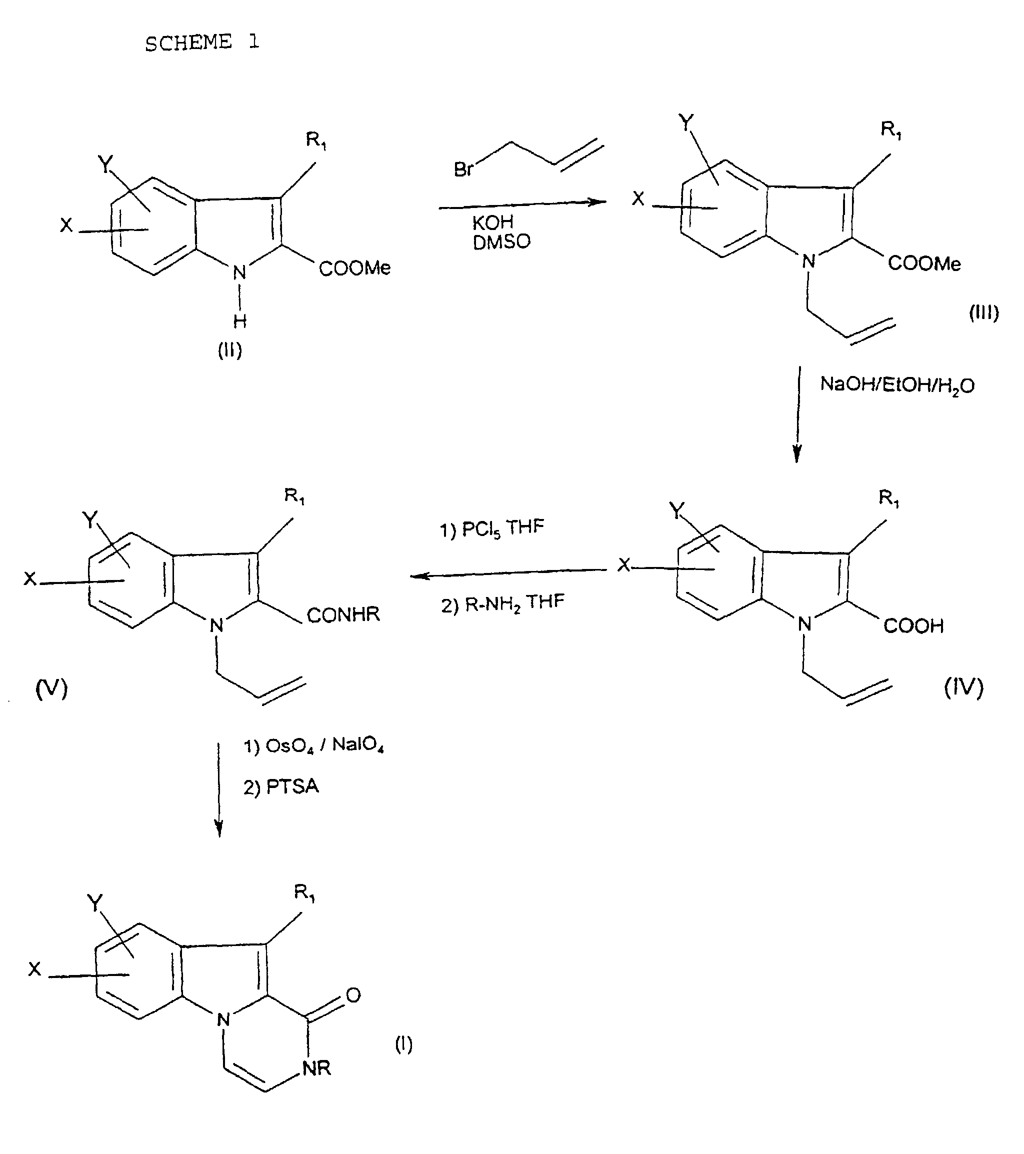

The compounds (II), which are known or can be prepared with known methods, are reacted with an allyl halide, for example allyl bromide, to give the compounds (III) which, after transformation of the ester group into amido group, are cyclized to compounds (I).

The cyclization reaction of compound (V) is carried out in two steps, in the first step operating in the presence of catalytic amounts of osmium tetroxide and of a stoichiometric amount of an alkali periodate, in the second one carrying out a dehydration in acidic medium, for example with para-toluenesulfonic acid in anhydrous organic solvents.

The other reactions used in the Scheme reported above may be effected according to the procedures conventionally used for N-alkylations, hydrolysis of ester groups, amidations and the like. For example, when the R, R₁, and R₂ groups contain substituents which interfere with the reactions of the Scheme above, suitable protective groups will be used and then removed according to conventional methods.

An alternative method for the synthesis of compounds of formula (I), which can be advantageously used when any easily oxidizable functional groups in the intermediates of formula (V) may interfere with the progress of the reaction with osmium tetroxide and alkali periodates, is that illustrated in the following scheme 2.

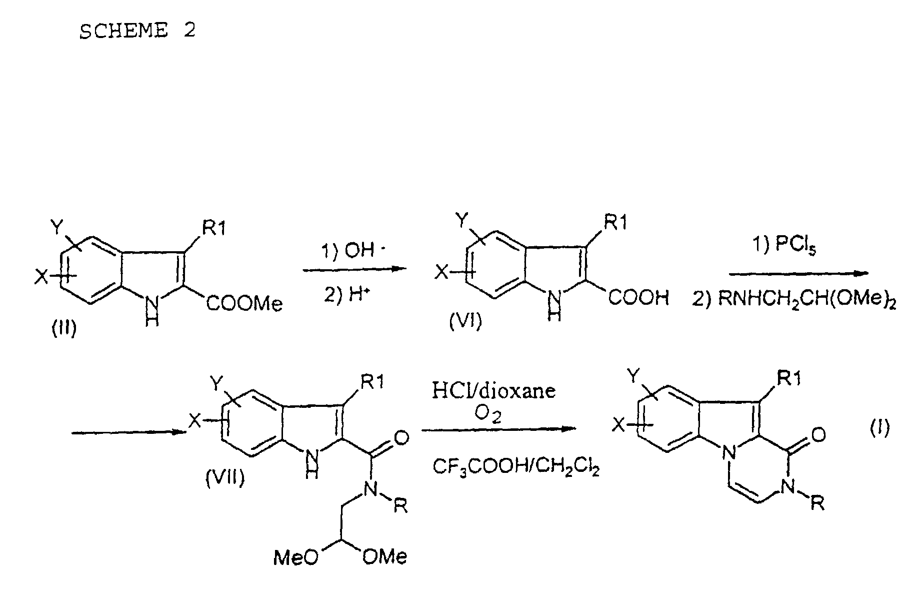

The compounds of formula (II) are hydrolysed to the corresponding 2-indolecarboxylic acids (VI) which are reacted with phosphorous pentachloride in an aprotic solvent and then with an aminoacetaldehyde derivative, such as 2-dimethylaminoacetaldehyde dimethylacetal, to give the compounds of formula (VII) which are then cyclized in the presence of acids, such as gas hydrochloric acid in dioxane or trifluoroacetic acid in methylene chloride, to compounds of formula (I)

An additional alternative method for the synthesis of compounds of formula (I) which can advantageously used when any easily oxidizable functional group is present in the R substituent of intermediates of formula (V) may interfere with the progress of the reaction with osmium tetroxide and alkali periodate, is the reaction of a compound of formula I, in which R is H, with an intermediate of formula VIII

R'-T (VIII)

in which R' has the same meaning as R, with the exception of H, and T represents an halogen such as chloro or bromine or a sulphonate ester such as the group mesilate (OSO₂CH₃), benzenesulphonate (OSO₂Ph) or p-toluenesulphonate.

The reaction is preferably conducted in the presence of at least one molar equivalent of a proton acceptor such an alkali or alkali-hearth hydroxide, alkoxide or hydride, such as, for example, NaOH, KOH, sodium methoxide, potassium tert-butoxide, sodium hydride, potassium hydride, in a solvent such as N,N-dimethylformamide, dimethylsulfoxide, N-methylpyrrolidinone and the like, working at a temperature between 0°C and the reflux temperature of the solvent. The reaction is preferably performed in DMSO or DMF, at room temperature, in the presence of at least one equivalent of potassium hydroxide.

The compounds of formula (I) in which Y is OR₂ and R₂ is hydrogen can be obtained by catalytic hydrogenation of the compounds of formula (I) in which X is OR₂ and R₂ is benzyl.

The compounds according to the invention have been pharmacologically tested against four human tumour cell lines: HT 29 (colon carcinoma), PC 3 (prostate carcinoma), H 460M (lung carcinoma), MCF-7 (breast carcinoma). Cells were incubated with the tested compound for 144 hours, then cytotoxicity was determined by using the MTT assay (Mosman, T. "Rapid Colorimetric Assay for Cellular Growth and Survival: Application to Proliferation and Cytotoxicity Assay"; J. Immunolog. Methods, (1983), 65, 66; Green, L.M., Rapid Colorimetric Assay for Cell Viability; Application to the Quantitation of Cytotoxic and Growth Inhibitory Lymphokines", J. Immunol. Methods, (1984), 70, 257-268).

The obtained data evidenced that the compounds according to the present invention have remarkable activity against solid tumors, in particular colon and lung tumors.

The compounds of the invention can be administered in doses ranging from 0.01 mg to 1 g / kg body weight daily. A preferred dosage regimen may range from about 1 mg to about 500 mg / kg body weight daily, using such unitary doses as to administer in 24 hours from about 70 mg to about 3.5 g of the active substance to a patient weighing about 70 Kg. Such dosage regimen may be adjusted in order to obtain a better therapeutical effect. For example, dosages may be adjusted in consideration of the therapeutical conditions of the patient.

The active compounds of the invention can be administered through the oral, intravenous, intramuscular or subcutaneous route.

The compounds of the invention may be administered, according to well-known therapeutical procedures, in combination with other agents used to induce the regression of tumors, in order to synergistically increase the antitumor effects of said compounds. Examples of compounds which can be used in combination with the compounds of the invention are cisplatin, carboplatin, doxorubicin, topotecan, taxol, taxotere, vincristine, 5-fluorouracil.

The pharmaceutical compositions according to the present invention contain therapeutically effective amounts of at least one compound of the invention in mixture with pharmaceutically acceptable excipients.

The oral compositions will generally include an inert diluent or an edible carrier and may be included in gelatin capsules or compressed into tablets. Other forms suitable for oral administration are capsules, pills, elixirs, suspensions or syrups.

The tablets, pills, capsules and similar compositions may contain the following ingredients (in addition to the active substance): a binder such as a microcrystalline cellulose, gum tragacanth or gelatin; an excipient such as starch or lactose; a disintegrating agent such as alginic acid, primogel, corn starch and the like; a lubricant such as magnesium stearate; a glidant such as colloidal silicon dioxide; a sweetening agent such as sucrose or saccharine or a flavoring agent such as peppermint, methyl salicylate or orange flavor. When the chosen composition is in form of capsules, it may contain in addition a liquid carrier such as a fatty oil. Other compositions may contain other various materials which modify the physical form, such as coating agents (for tablets and pills) such as sugar or shellac. The materials used in the preparation of the compositions should be pharmaceutically pure and not toxic at the employed dosages.

For the preparation of pharmaceutical compositions for the parenteral administration, the active ingredient may be incorporated into solutions or suspensions, which may include in addition the following components: a sterile diluent such as water for injection, saline solution, oils, polyethylene glycols, glycerin, propylene glycol or other synthetic solvents; antibacterial agents such as benzyl alcohol; antioxidants such as ascorbic acid or sodium bisulphite; chelating agents such as ethylenediaminotetraacetic acid; buffers such as acetates, citrates or phosphates and agents for adjusting the solution tonicity such as sodium chloride or dextrose. The parenteral preparation may be included in ampoules, disposable syringes or glass or plastic vials.

The invention is further described by the following examples.

### Preparation 1

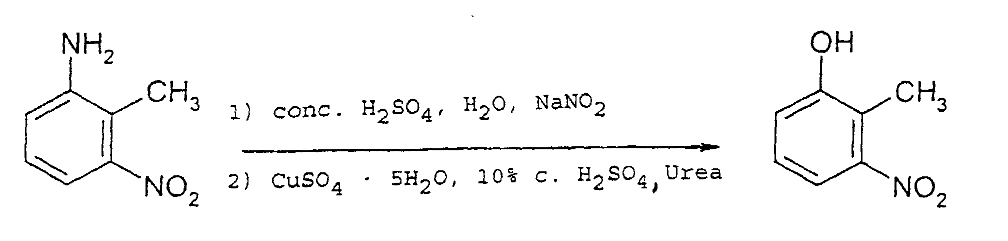

952.0 mg of 6-nitro-2-aminotoluene are suspended in 15 mL of water, then 3 mL of conc. H₂SO₄ and 10 mL of 10% H₂SO₄ are added thereto. When the amine is almost completely dissolved, the solution is cooled to 0°C and added with 561.1 mg (8.132 mmoles) of NaNO₂ dissolved in 4 mL of H₂O. The mixture is stirred for 1h30', added with 468.8 mg (1.878 mmoles) of CuSO₄·5H₂O and 364.5 mg (6.070 mmoles) of Urea, then heated to 90°C for 2h30' and finally left at room temperature overnight.

The straw-yellow precipitate is filtered and dissolved in 35 mL of 5% NaOH, the residue is filtered and the solution is acidified to acid pH with 5% HCl (36 mL).

The precipitate is filtered and dried under vacuum, to obtain 680 mg of a pale yellow solid.

The reaction yield is 71%.
¹H-NMR (200 MHz, CDCl₃)
δ (ppm): 7.43 (d, 1H, H₃, J_{3,2} = 7.80 Hz); 7.18 (t, 1H, H₂); 7.00 (d, 1H, H₁, J_{1,2} = 7.46 Hz); 5.40-4.50 (bs, 1H, OH); 2.40 (s, 3H, CH₃)
m.p.: 133-135°C.

### Preparation 2

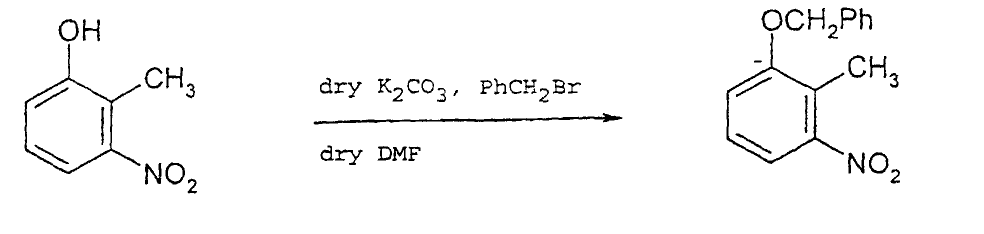

13.41 g (0.0876 mols) of the derivative of Preparation 1 are dissolved in dry DMF (88 mL) in a round-bottom flask under inert argon atmosphere, then added with 12.12 g (0.0877 mols) of dry K₂CO₃ and kept under stirring at room temperature for 30 minutes. Afterwards, 11.2 mL (0.0943 mols) of benzyl bromide are added heating to 60°C for 4h.

The reaction is monitored by TLC (Hexane/Ethyl acetate 8:2).

The inorganic salts are filtered off, washing with DMF (50 mL) and the solvent is evaporated off to an about 40 mL volume. The resulting oily residue is filtered through silica (packed with Hexane/Ethyl acetate 9:1), to obtain 14.62 g of straw-yellow solid product.

The reaction yield is 68.6%.
¹H-NMR (200 MHz, CDCl₃)
δ (ppm): 7.43-7.08 (m, 8H, H₁, H₂, H₃, hydrogens on Ph); 5,14 (s, 2H, CH₂Ph); 2.43 (s, 3H, CH₃).

### Preparation 3

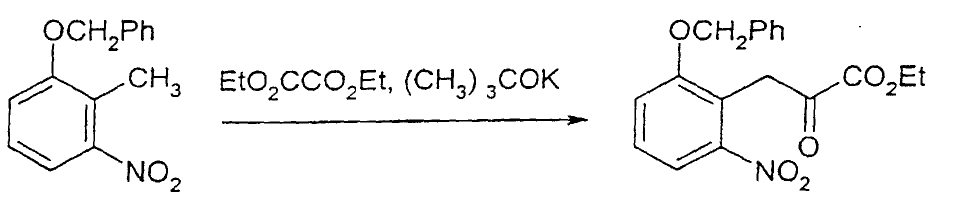

8.47 g (0.0755 mols) of potassium tert-butoxide and 70 mL of diethyl oxalate are mixed in a round-bottom flask under inert argon atmosphere, stirring until t-butoxide is dissolved. The yellowish solution is added with 14.62 g (0.060 mols) of the derivative of Preparation 2 and heated at 60°C for 2h.

The reaction is monitored by TLC (Hexane/Ethyl acetate 8:2).

The resulting purple solution is added with 120 mL of ethyl ether and pH is adjusted to neutral with acetic acid (4.4 mL); then the mixture is added with water and extracted with ether (80 mL *3). The organic solution is dried over sodium sulfate and the solvent is evaporated off.

The resulting residue is directly used for the subsequent reaction.

### Preparation 4

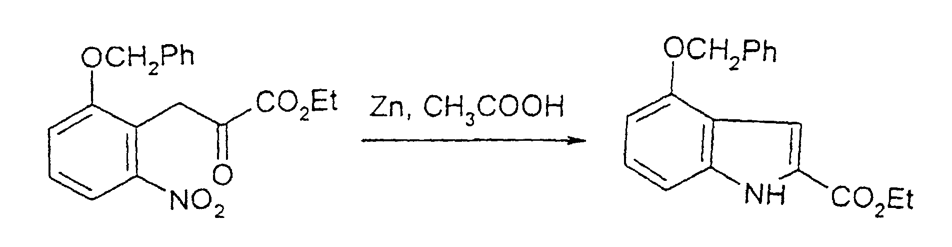

21.0 g of the derivative of Preparation 3 are dissolved in 227 mL of 98% acetic acid in a round-bottom flask, then added with 10.1 mL of H₂O and heated under reflux. After that, 29.08 g (0.445 mols) of powder zinc are added, stirring for 30 minutes.

The reaction is monitored by TLC (Hexane/Ethyl acetate 8:2).

The zinc salts are filtered off and washed with ethanol (30 mL *3) and acetic acid (25 mL *2).

The combined filtered solutions are added with water (250 mL) to precipitate the product, which is filtered and washed with some water.

The resulting product requires no further purifications.

The reaction yield is 76.6% on the two steps.
¹H-NMR (200 MHz, CDCl₃)
δ (ppm) : 8.95 (bs, 1H, HN); 7.53-7.33 (m, 6H, H₅, H on Ph); -7.22 (t, 1H, H₈); 7.03 (d, 1H, H₇); 6.57 (d, 1H, H₉); 5.23 (s, 2H, CH₂Ph); 4.41 (q, 2H, CH₂ of COOEt); 1.494 (t, 3H, CH₃ of COOEt).

### Preparation 5

### Catalytic hydrogenation of ethyl 4-benzyloxyindole-2-carboxylate to give ethyl 4-hydroxyindole-2-carboxylate

A solution of ethyl 4-benzyloxyindole-2-carboxylate of Preparation 4 (122,3 mg) in MeOH/THF 2/1 is hydrogenated under atmospheric pressure and at room temperature in the presence of 10% palladium on charcoal (83,3 mg). After 1h45' the absorption of hydrogen ceases. The catalyst is filtered off and washed with THF (10 mL). The combined filtrates are concentrated to dryness by distillation under reduced pressure to give a crude which is purified by column chromatography (silica; eluent petroleum ether /ethyl acetate 8/2), to obtain 71.9 mg of ethyl 4-hydroxyindole-2-carboxylate.

### Preparation 6

### Alkylation of ethyl 4-hydroxyindole-2-carboxylate with benzyl bromide to give ethyl 4-benzyloxyindole-2-carboxylate.

A solution of ethyl 4-hydroxyindole-2-carboxylate (71.9 mg) in DMSO (1 mL) stirred under nitrogen atmosphere is added with K₂CO₃ (103.0 mg) and after 10' with benzyl bromide (0.05 mL). The reaction mixture is left under stirring for 2h, then diluted with MeOH (1 mL) and after 5' filtered to remove the solid material present, washing the solid on the filter with CH₂Cl₂ (5 mL). The combined filtrates are concentrated to small volume and the resulting residue is purified by column chromatography (silica, petroleum ether / ethyl acetate 9/1), to obtain 32.2 mg of ethyl 4-benzyloxyindole-2-carboxylate.
m.p. 166-168°C (acetic acid/water).
Analogously are prepared:
ethyl 4- (4-chlorobenzyloxy)indole-2-carboxylate;
ethyl 4- (4-methoxybenzyloxy)indole-2-carboxylate;
ethyl 4-(4-trifluoromethylbenzyl)oxyindole-2-carboxylate.

### Preparation 7

### Hydrolysis of ethyl 4-benzyloxyindole-2-carboxylate to 4-benzyloxyindole-2-carboxylic acid.

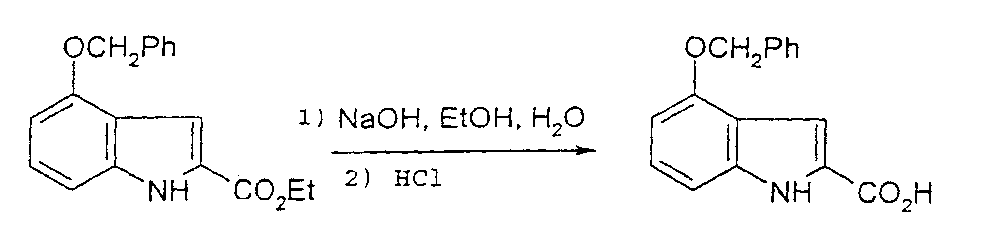

13.55 g (0.0459 mols) of the derivative of Preparation 4 are dissolved in a round-bottom flask in 68 mL of ethanol. The mixture is heated to 50°C and added with 2.31 g (0.0578 mols) of NaOH dissolved in 40 mL of distilled water, heating to 80-90°C for 2h.

The reaction is monitored by TLC (Hexane / Ethyl acetate 8:2 and CH₂Cl₂/MeOH 9:1).

The mixture is acidified to acid pH with 5% HCl (42.0 mL) and the precipitated acid is filtered. The resulting whitish product requires no further purifications. The reaction yield is quantitative.

### Preparation 8

### N-(2,2-dimethoxyethyl)-N-methyl-4-benzyloxyindole-2-carboxyamide

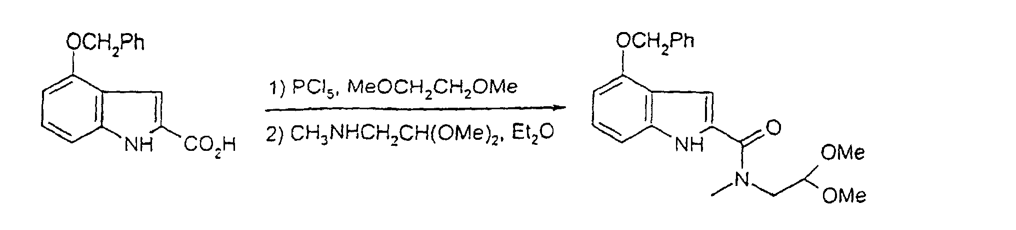

14.00 g (0.0524 mols) of the crude derivative of Preparation 7 are dissolved in 88 mL of 1,2-dimethoxyethane in a round-bottom flask under inert argon atmosphere, 13.115 g (0.0629 mols) of PCl₅ are added and the mixture is stirred at room temperature for 3h; then heated to 40°C for 1h. The solvent is evaporated off and the residue is taken up with 30 mL of 1,2-dimethoxyethane then, still under inert atmosphere, is cooled to 0°C and added with 19 mL (0.148 mols) of 2-(methylamino)acetaldehyde dimethylacetal dissolved in 100 mL of ethyl ether. The mixture is stirred at 0°C for ten minutes, then warmed to room temperature and left under stirring overnight.

The reaction is monitored by TLC (Hexane/Ethyl acetate 1:1 and CH₂Cl₂/MeOH 9:1).

The precipitated product is filtered, washing thoroughly with ether. The resulting product still contains traces of the starting 2-dimethylaminoacetaldehyde dimethylacetal. The product however is not subjected to further purifications.
¹H-NMR (300 MHz, CDCl₃)
δ (ppm); 9.45 (bs, 1H, HN); 7.51-7.02 (m, 8H, H₅, H₇, H₈, H on Ph); 6.57 (d, 1H, H₉); 5.22 (s, 2H, CH₂Ph); 4.65 (t, 1H, H₁); 3.75 (bs, 2H, H₂); 3.45 (bs, 9H, NCH₃, OCH₃).

Analogously are prepared the following compounds, by reaction between the suitable indole-2-carboxylic. acids and aminoacetaldehydes dialkyl acetals:
N-(2,2-dimethoxyethyl)-5-benzyloxyindole-2-carboxyamide,
m.p. 134-135°C;
N-(2,2-dimethoxyethyl)-indole-2-carboxyamide, m.p. 102-103°C;
¹H-NMR (DMSO-d6, ppm): 11.5 (s, 1H); 7.61 (d, 1H, J = 8 Hz); 7.42 (d, 1H, J = 8 Hz); 7.19 (dd, 1H, 8 Hz, 8 Hz); 7.04 (dd, 1H, J = 8 Hz, 8 Hz), 6.90 (s, 1H); 4.60 (t, 1H); 3.8-3.5 (br, 2H); 3.35 (2 s, 9H);

| Elemental analysis: | | | |
|---|---|---|---|
| Calculated for C₁₄H₁₈N₂O₃ | C = 64.10, | H = 6.92, | N = 10.68 |
| Found: | C = 64.10, | H = 6.94, | N = 10.67 |

N-(2,2-dimethoxyethyl)-5-benzyloxyindole-2-carboxyamide,
m.p. 145-146°C;
¹H-NMR (DMSO-d6, ppm): 11.6 (s, 1H); 8.5 (t, 1H, J = 5.5 Hz); 7.58-7.25 (m, 6H); 7.08 (d, 1H, J = 7 Hz); 7.05 (s, 1H); 6.60 (dd, 1H, J = 7Hz, J = 1 Hz); 5.2 (s, 2H); 4.50 (t, 1H, J = 5.5 Hz); 3,37 (dd, 2H, J = 5.5 Hz); 3.29 (s, 6H);
N-(2,2-dimethoxyethyl)-N-methyl-4-methoxyindole-2-carboxyamide, m.p. 104-105°C;
N-(2,2-dimethoxyethyl)-4-methoxyindole-2-carboxyamide,
m.p. 165-166°C;

### Example 1

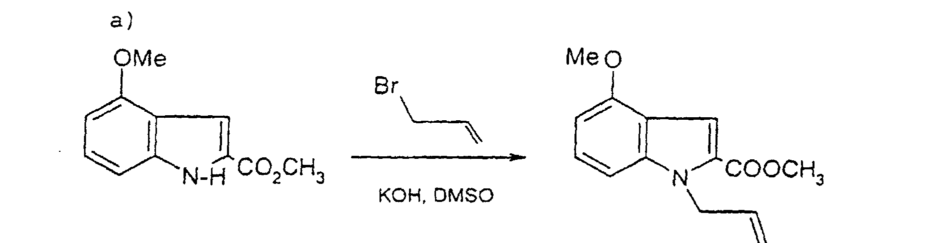

1.0780 g (19.209 mmoles) of KOH (ground pellets) are suspended in 50 mL of dry DMSO in a round-bottom flask under inert argon atmosphere; the mixture is stirred for five minutes then added with 0.992 g (4.834 mmoles) of methyl 4-methoxy-indole-2-carboxylate stirring for 40'. The reaction mixture is then added with 0.826 mL (9.764 mmoles) of allyl bromide, stirring at room temperature until completion of the reaction.

One hour after, by checking with TLC (Hexane/Ethyl acetate 8:2) the reaction is completed; the mixture is added with 5% HCl to neutral pH, then with water and is finally extracted with ethyl acetate. The organic phase is dried over anhydrous sodium sulfate and evaporated.

The resulting residue is purified by flash chromatography (eluent Hexane/Ethyl acetate 98:2), to yield 802.9 mg of methyl-4-methoxy-1-allyl-indole-2-carboxylate as a colourless liquid.

The reaction yield is 68%.
1H-NMR (300 MHz, CDCl₃)
δ (ppm) : 7.45 (s, 1H, H5) ; 7.24 (t, 1H, H₈); 6.95(d, 1H, H₇, J_{7.8} = 8.45 Hz); -6.50 (d, 1H, H₉, J_{9.8} = 7.78 Hz; 6.04-5.92 (m, 1H, H₂); 5.20-5.19 (m, 2H, H₃); 5.10-5.06 (m, 1H, H₁ₐ); 4.91-4.86 (m, 1H,H_{1b}); 3.95 (s, 3H, OCH₃ Ph); 3.79 (s, 3H, OCH₃ of COOMe).

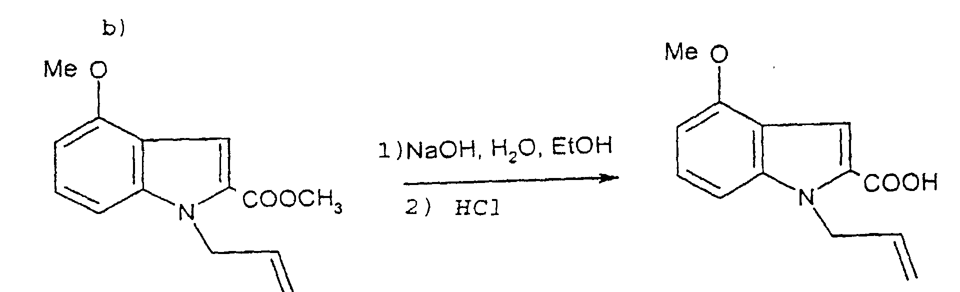

1.9303 g (7.870 mmoles) of the compound from a) are dissolved in 9 mL of absolute EtOH and said solution is added with 652.1 mg (15.740 mmoles) of NaOH dissolved in 12 mL of H₂O, heating at 60-70°C for 2h.

The reaction is followed by TLC (Hexane/Ethyl acetate 9:1 and CH₂Cl₂MeOH 9:1).

The reaction mixture is extracted with CH₂Cl₂, washing thoroughly the organic phase with H₂O, then the aqueous phase is acidified to acid pH with 9% HCl (7 mL), to obtain 11.6 mg of 1-allyl-4-methoxyindole-2-carboxylic acid as a white solid.

The product requires no further purifications.

The reaction yield is 86.8%.
1H-NMR (200 MHz, CDCl₃)
δ (ppm) : 7.60 (s, 1H, H₅); 7.28 (t, 1H, H₈); 7.26 (s, 1H, OH of COOH); 6.97 (d, 1H, H₇, J_{7.8} = 8.44 Hz); -6.52 (d, 1H, H₉, J_{9.8} = 7.73 Hz; 6.10-5.92 (m, 1H, H₂); 5.23-5-19 (m, 2H, H3); 5.12 (dd, 1H, H₁ₐ H_{1a,3} = 10.23 Hz, J_{1a,1b} = 1.13 Hz); 4.92 (dd, 1H, H_{1b} H_{1b,3} = 16.09 Hz, J_{1a,1b} = 1.15 Hz); 3.95 (s, 3H, OCH₃).

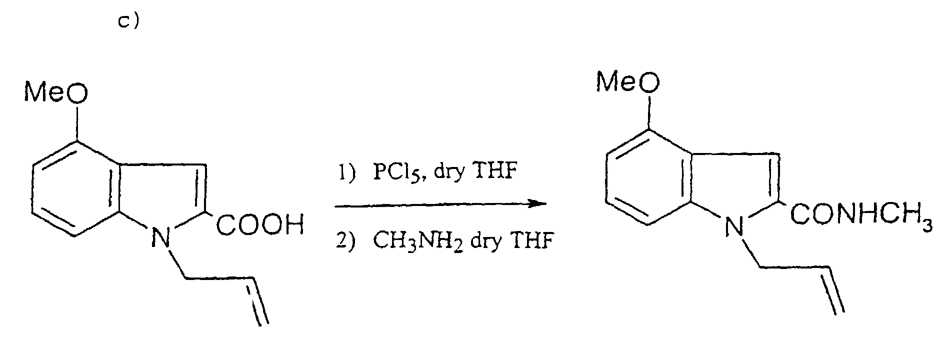

388.5 mg (1.680 mmoles) of the derivative obtained in b) are dissolved in 2.8 mL of dry THF in a round-bottom flask under inert argon atmosphere, after that 424.5 mg (2.037 mmoles) of PCl₅ are added. The mixture is stirred at room temperature for 2h 30', then heated to 40-50°C for 1h. The solvent is evaporated off and the residue is redissolved in 1.2 mL of dry THF. After cooling to 0°C, the reaction mixture is added with 2.20 mL of MeNH₂ in solution of 1.52 M THF (3.344 mmoles) stirring for 10 minutes, then warmed to room temperature overnight.

The reaction is monitored by TLC (CH₂Cl₂/MeOH 9:1).

The reaction mixture is added with water and extracted with CH₂Cl₂, the organic phase is dried over anhydrous sodium sulfate and evaporated.

The resulting residue is purified by flash chromatography (eluent Hexane/Ethyl acetate 6:4, plus 0.5% triethylamine for packing the column), to yield 232.6 mg of 1-allyl-4-methoxyindole-2-carboxylic acid methylammide as a white solid.

The reaction yield is 58%.
¹H-NMR (200 MHz, CDCl₃)
δ (ppm) : 7.21 (t, 1H, H8); 6.99-6.95 (m, 2H, H₇, H₅); 6-52 (d, 1H, H₉, J_{9.8} = 7.71 Hz); 6.17-5.92 (m, 2H, H₂, NH); 5.20 (d, 2H, H₃); 5.11-5.06 (m, 1H, H₁ₐ H_{1a,2} = 9.28 Hz, J_{1a,1b} = 1.34 Hz); 4.96-4.87 (m, 1H, H_{1b} H_{1b,2} = 16.55 Hz, J_{1b,1a} = 1.35 Hz); 3.95 (s, 3H, OCH₃); 3.00 (d, 3H, NCH₃ J_{NH,MeN} ≅ 52 Hz).

Mass spectrum (C.I.) m/z : 245 (M+1); 246 (M+2).

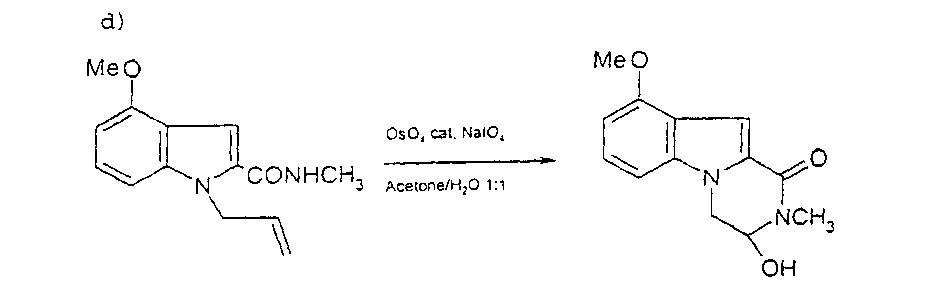

38.4 mg (0.157 mmoles) of the derivative obtained in c) are dissolved in 8 mL of Acetone/H2O 1:1 in a round-bottom flask, then cooled to 0°C and added with 0.4 mL (0.00786 mmoles) of osmium tetroxide in tert-butanol (5 mg/mL solution). The mixture is stirred for 5 minutes and added with 108.8 mg (0.509 mmoles) of sodium metaperiodate.

The reaction is monitored by TLC (AcOEt).

After 5 days the reaction is complete: the reaction mixture is added with aqueous Na₂S₂O₃ until it turns yellow, then with water and extracted with CH₂Cl₂. The organic phase is dried over anhydrous sodium sulfate and evaporated.

The resulting residue is purified by flash chromatography (eluent Hexane/Ethyl acetate 1:9), to yield 19.0 mg of 8-methoxy-2-methyl-1,2,3,4-tetrahydro-3-hydroxy-1-oxo-pyrazino [1,2-a]indole as a white solid.

The reaction yield is 49.1%.
¹H-NMR (200 MHz, CD₃COCD₃)
δ (ppm) : 7.25-7.00 (m, 3H, H₈, H₅, H₇,); -6.57(d, 1H, H₉); 5.47-5.41 (m, 1H, H₁); 4.52 (dd, 1H, H₂ₐ); 4.29 (dd, 1H, H_{2b}); 3.95 (s, 3H, OCH₃); 3.15 (s, 3H, NCH₃).

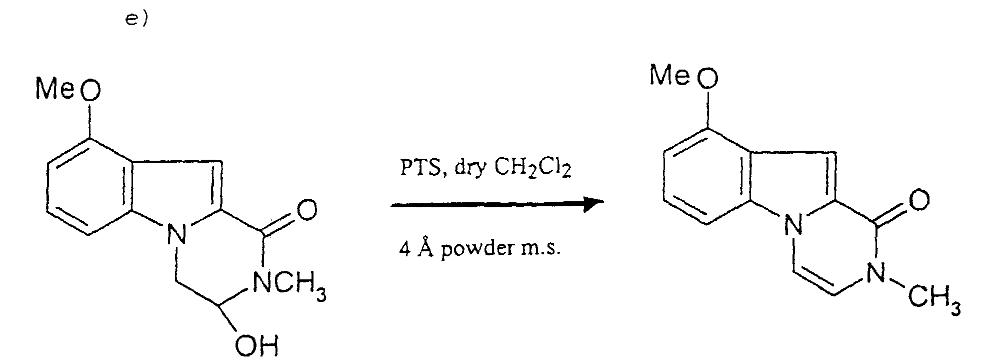

83.2 mg (0.338 mmoles) of the derivative obtained in d) are dissolved in dry CH₂Cl₂ (10 mL) in a round-bottom flask under inert argon atmosphere, added with 67.7 mg (0.356 mmoles) of p-toluenesulfonic acid and 876 mg of 4 Å powder molecular sieves. The reaction mixture is stirred at room temperature overnight.

The reaction is monitored by TLC (Ethyl acetate).

The mixture is filtered through Celite, washing with methylene chloride, and the solvent is evaporated off.

The residue is purified by flash chromatography (eluent Hexane/Ethyl acetate 1:9) to yield 38.4 mg of 8-methoxy-2-methyl-1,2-dihydro-1-oxo-pyrazino[1,2-a]indole as a white solid.

The reaction yield is 50%.
m.p. 178-179 °C.
¹H-NMR (200 MHz, CDCl₃)
δ (ppm) : 7.51 (s, 1H, H₅); 7.36-7.19 (m, 3H, H₇, H₈, H₁ o H₂); 6.65 (d, 1H, H₁ o H₂); 6.39 (d, 1H, H₉); 3.95 (s, 3H, OCH₃); 3.50 (s, 3H, CH₃N).
Mass spectrum (C.I.) m/z: 229 (M+1).

### Example 2

Trifluoroacetic acid (0.093 mL) is added under nitrogen atmosphere to a solution of N-(2,2-dimethoxyethyll)-4-benzyloxyindole-2-carboxyamide (148 mg) in CH₂Cl₂ (1.5 mL). The reaction mixture is left at room temperature for 3h,30', then diluted with CH₂Cl₂ (3 mL) and neutralized with NH₄OH (0.74 mL of a 32% solution diluted with 0.76 mL of water). The organic phase is separated, the aqueous phase is re-extracted with CH₂Cl₂ (2 x 10 mL). The combined organic phases are dried and evaporated to dryness. The resulting residue is purified by flash chromatography (silica).

The desired product is eluted with ethyl acetate / n-hexane 6/4, to give 8-benzyloxy-1,2-dihydro-1-oxo-pyrazino[1,2-a]indole (19.6 mg), m.p. 259-262°C.

¹H-NMR(DMSO-d6, ppm): 10.57 (s, 1H); 7.77 (d, 1H, J = 5.6 Hz); 7.65-7.25 (m, 7H); 7.22 (s, 1H); 6.84 (d, 1H, J = 7.87 Hz); 6.64 (dd, 1H, J = 5.6 Hz, J = 5.6 Hz); 5.3 (s, 2H).

Further elution with ethyl acetate / isopropanol /methylene chloride 10/1/0.6 yields a second product which is 5-benzyloxy-lH-pirido[3,4-b]indol-1-one.

### Example 3

The following 1,2-dihydro-1-oxo-pyrazino[1,2-a]indoles are obtained either using in the procedures described in Example 1 the methyl or ethyl esters of the suitable indole-2-carboxylic acids (Example la) and the suitable amines (Example 1c), or using in the procedures described in Example 2 the suitable N-(2,2-dialkoxyethyl)-indole-2-carboxyamides:
2-methyl-1,2-dihydro-1-oxo-pyrazino[1,2-a]indole; m.p. 143-145°C; 1H-NMR (DMSO-d6, ppm): 8.05 (d, 1H, J = 7.5 Hz; 7.92 (d, 1H, J = 6.0 Hz); 7.81 (d, 1H, J = 7,5 Hz); 7.40 (dd, 1H, J = 7.5 Hz, J = 7.5 Hz); 7.28 (dd, 1H, J = 7.5 Hz, J = 7.5 Hz); 7.22 (s, 1H); 6.70 (d, 1H, J = 6 Hz); 3.45 (s, 3H).

| Elemental analysis: | | | |
|---|---|---|---|
| Calculated for C₁₂H₁₀N₂O | C = 72.71, | H = 5.09, | N = 14.19 |
| Found: | C = 71.91, | H = 5.02, | N = 14.01 |

1,2-dihydro-1-oxo-pyrazino[1,2-a]indole, m.p. 263-264°C;
2-benzyl-1,2-dihydro-1-oxo-pyrazino[1,2-a]indole;
2-(2-dimethylaminoethyl)-1,2-dihydro-1-oxo-pyrazino[1,2-a]-indole, m.p. 120-121°C; hydrochloride, m.p. 266-272°C;
2-(3-hydroxypropyl)-1,2-dihydro-1-oxo-pyrazino[1,2-a]-indole ;
8-benzyloxy-2-methyl-1,2-dihydro-1-oxo-pyrazino[1,2-a]-indole, m.p. 171-172°C;
¹H-NMR (DMSO-d6, ppm): 7.87 (d, 1H, J = 6.25 Hz); 7.70-7.25 (m, 7H); 7.20 (s, 1H); 6.91 (d, 1H, J = 6.25 Hz); 6.85 (d, 1H, J = 8.13 Hz); 5.32 (s, 2H); 3.42 (s, 3H).

| Elemental analysis: | | | |
|---|---|---|---|
| Calculated for C₁₉H₁₆N₂O₂ | C = 74.98, | H = 5.30, | N = 9.20 |
| Found: | C = 74.11, | H = 5.25, | N = 9.09 |

7-benzyloxy-2-methyl-1,2-dihydro-1-oxo-pyrazino-[1,2-a]indole, m.p. 193-194°C;
7-benzyloxy-2-(2-pyridylmethyl)-1,2-dihydro-1-oxo-pyrazino-[1,2-a]indole;
7-benzyloxy-2-(3-pyridylmethyl)-1,2-dihydro-1-oxo-pyrazino-[1,2-a]indole;
7-benzyloxy-2-(4-pyridylmethyl)-1,2-dihydro-1-oxo-pyrazino-[1,2-a]indole;
7-benzyloxy-2-benzyl-1,2-dihydro-1-oxo-pyrazino[1,2-a]-indole;
7-benzyloxy-2-(2-phenylethyl)-1,2-dihydro-1-oxo-pyrazino-[1,2-a]indole;
7-benzyloxy-2-phenyl-1,2-dihydro-1-oxo-pyrazino[1,2-a]-indole;
7-benzyloxy-2-(3-chloro-2-cyanophenyl)-1,2-dihydro-1-oxo-pyrazino[1,2-a]indole;
7-benzyloxy-2-(3-fluoro-2-cyanophenyl)-1,2-dihydro-1-oxo-pyrazino[1,2-a]indole;
7-benzyloxy-2-(3-chloro-5-trifluoromethylpyridyl)-1,2-dihydro-1-oxo-pyrazino[1,2-a]indole;
7-benzyloxy-2-(2-thienylmethyl)-1,2-dihydro-1-oxo-pyrazino-[1,2-a]indole;
7-benzyloxy-2-(2-hydroxy-3-methoxybenzyl)-1,2-dihydro-1-oxo-pyrazino[1,2-a]in-dolo;
7-benzyloxy-2-(2-thienyl)-1,2-dihydro-1-oxo-pyrazino[1,2-a]indole;
7-benzyloxy-2-(4-methylphenyl)-1,2-dihydro-1-oxo-pyrazino-[1,2-a]indole;
8-methoxy-2-methyl-1,2-dihydro-1-oxo-pyrazino[1,2-a]indole;
8-methoxy-1,2-dihydro-1-oxo-pyrazino[1,2-a]indole,
m.p. > 300°C.
7-benzyloxy-2-methyl-1,2-dihydro-1-oxo-pyrazino[1,2-a]-indole;
7-benzyloxy-1,2-dihydro-1-oxo-pyrazino[1,2-a]indole;
7-methoxy-2-methyl-1,2-dihydro-1-oxo-pyrazino[1,2-a]indole;
7-methoxy-1,2-dihydro-1-oxo-pyrazino[1,2-a]indole;
7-nitro-2-methyl-1,2-dihydro-1-oxo-pyrazino[1,2-a]indole;
7-nitro-1,2-dihydro-1-oxo-pyrazino[1,2-a]indole;
5-nitro-2-methyl-1,2-dihydro-1-oxo-pyrazino[1,2-a]indole;
5-nitro-1,2-dihydro-1-oxo-pyrazino[1,2-a]indole;
7-methylsulfonyl-2-methyl-1,2-dihydro-1-oxo-pyrazino[1,2-a]indole;
7-methylsulfonyl-1,2-dihydro-1-oxo-pyrazino[1,2-a]indole;
7-methyl-2-methyl-1,2-dihydro-1-oxo-pyrazino[1,2-a]indole;
7-methyl-1,2-dihydro-1-oxo-pyrazino[1,2-a]indole;
7-trifluoromethoxy-2-methyl-1,2-dihydro-1-oxo-pyrazino[1,2-a]indole;
7-trifluoromethoxy-1,2-dihydro-1-oxo-pyrazino[1,2-a]indole;
7-fluoro-2-methyl-1,2-dihydro-1-oxo-pyrazino[1,2-a]indole;
7-fluoro-1,2-dihydro-1-oxo-pyrazino[1,2-a]indole;
7-chloro-2-methyl-1,2-dihydro-1-oxo-pyrazino[1,2-a]indole;
7-chloro-1,2-dihydro-1-oxo-pyrazino[1,2-a]indole;
7-hydroxy-2-methyl-1,2-dihydro-1-oxo-pyrazino[1,2-a]indole;
7-hydroxy-1,2-dihydro-1-oxo-pyrazino[1,2-a]indole;
6-methoxy-2-methyl-1,2-dihydro-1-oxo-pyrazino[1,2-a]indole;
6-methoxy-1,2-dihydro-1-oxo-pyrazino[1,2-a]indole;
6,8-dimethoxy-2-methyl-1,2-dihydro-1-oxo-pyrazino[1,2-a]-indole;
6,8-dimethoxy-1,2-dihydro-1-oxo-pyrazino[1,2-a]indole;
7-methoxy-2-methyl-1,2-dihydro-1-oxo-pyrazino[1,2-a]indole; 7-methoxy-1,2-dihydro-1-oxo-pyrazino[1,2-a]indole;
7-bromo-2-methyl-1,2-dihydro-1-oxo-pyrazino[1,2-a]indole;
7-bromo-1,2-dihydro-1-oxo-pyrazino[1,2-a]indole;
7-benzyloxy-6-methoxy-2-methyl-1,2-dihydro-1-oxo-pyrazino-[1,2-a]indole;
6-methoxy-7-benzyloxy-1,2-dihydro-1-oxo-pyrazino[1,2-a]-indole;
7-ethyl-2-methyl-1,2-dihydro-1-oxo-pyrazino[1,2-a]indole;
7-ethyl-1,2-dihydro-1-oxo-pyrazino[1,2-a]indole;
7-bromo-9-methyl-1,2-dihydro-1-oxo-pyrazino[1,2-a]indole;
2,9-dimethyl-1,2-dihydro-1-oxo-pyrazino[1,2-a]indole;
8-benzyloxy-2,9-dimethyl-1,2-dihydro-1-oxo-pyrazino[1,2-a]-indole.

### Example 4

Pulverised potassium hydroxide (1.005 g) is suspended in dry dimethylsulfoxide (4 ml). After stirring for 5 minutes, 1,2-dihydro-1-oxo-pyrazino[1,2-a]indole (0.50 g) is added followed by, after 30 min, 2-dimethylaminoethylchloride hydrochloride (0.899 g). After stirring for 6 hours, the reaction is quenched with water and the formed precipitate is recovered by filtration. This solid is taken up in boiling tert-butyl methyl ether and the suspension filtered while hot. The insoluble material is discarded. The filtrate is concentrated to dryness and the residue is recrystallised two times from tert-butyl methyl ether. A final purification from silica gel column chromatography (eluent: CH₂Cl₂/MeOH 9/1) affords 2-(2-dimethylaminoethyl)-1,2-dihydro-1-oxo-pyrazino[1,2-a]indole (154 mg), m.p. 120-121°C.

In a similar way is prepared the following compound:
7-benzyloxy-2-(2-dimethylaminoethyl)-1,2-dihydro-1-oxo-pyrazino[1,2-a]indole, m.p. 265-268°C.

## Claims

1. Compounds of formula (I) 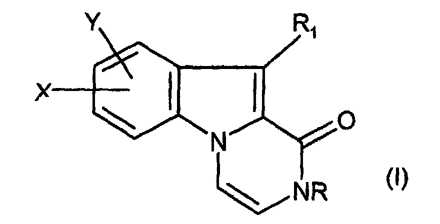 wherein:
R is hydrogen; C₁-C₆ alkyl optionally substituted with one or more primary, secondary or tertiary amino groups, hydroxy or carboxy groups; C₅-C₆ cycloalkyl; aralkyl; aryl; heteroaryl; heteroaralkyl;
R₁ is hydrogen; C₁-C₆ alkyl optionally substituted with one or more primary, secondary or tertiary amino groups, hydroxy or carboxy groups; C₅-C₆ cycloalkyl; aralkyl; halogen;
Y is hydrogen or a -O-R₂ group;
R₂ is hydrogen; C₁-C₆ alkyl optionally substituted with one or more primary, secondary or tertiary amino groups, hydroxy or carboxy groups; C₅-C₆ cycloalkyl; aralkyl; aryl; heteroaryl; heteroarylalkyl; C₁-C₆ acyl;
X is one or more groups independently selected from hydrogen, C₁-C₆ alkyl, hydroxy, C₁-C₄ alkoxy, C₁-C₃ haloalkoxy, amino, C₁-C₃ alkylamino, C₁-C₃-acylamino, C₁-C₃- alkylsulfonylamino, halogen, nitro, cyano, carboxy, C₁-C₃ alkoxycarbonyl, sulfonyl, C₁-C₃-alkylsulfonyl, aminosulfonyl, C₁-C₃- alkylaminosulfonyl, trifluoromethyl;
and wherein:
aralkyl is benzyl or phenethyl;
aryl is phenyl, naphthyl or phenyl substituted with one or two substituents selected from C₁-C₆ alkyl, hydroxy, C₁-C₄ alkoxy, C₁-C₃ haloalkoxy, amino, C₁-C₃ alkylamino, C₁-C₃ acylamino, C₁-C₃-alkylsulfonylamino, halogen, nitro, cyano, carboxy, C₁-C₃ alkoxycarbonyl, sulfonyl, C₁-C₃-alkylsulfonyl, aminosulfonyl, C₁-C₃-alkylaminosulfonyl, trifluoromethyl;
heteroaryl is furyl, thienyl, imidazolyl, benzothienyl, benzofuryl, pyridyl, indolyl, pyrimidyl, thiazolyl;
heteroarylalkyl is pyridylmethyl, thienylmethyl, furylmethyl, isoquinolylmethyl.

2. Compounds as claimed in claim 1, wherein the OR₂ group is at the 8- position of the 1,2-dihydro-1-oxo-pyrazino-[1,2-a]indole heterocyclic system.

3. Compounds as claimed in claim 1 or 2 wherein R is C₁-C₆ alkyl, R₁ is hydrogen, X is hydrogen and R₂ is C₁-C₆ alkyl, aralkyl or aryl, wherein aralkyl and aryl are as defined in claim 1.

4. Pharmaceutical compositions containing as active ingredient a compound of claims 1 - 3 in mixture with a suitable carrier.

5. Compositions as claimed in claim 4, as combined preparations for the sequential or simultaneous administration, containing, in addition to a compound of claims 1 - 3, an antitumor drug with synergistic action.

6. The use off the compounds of claims 1 - 3 for the preparation of antitumor medicaments.

## Patentansprüche

1. Verbindung der Formel (I) 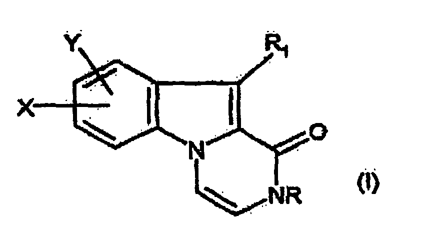 worin:
R Wasserstoff; (C₁-C₆)Alkyl, wahlweise substituiert mit einer oder mehreren primären, sekundären, oder tertiären Aminogruppen, Hydroxyoder Carboxygruppen; (C₅-C₆)Cycloalkyl; Aralkyl; Aryl; Heteroaryl; Heteroaralkyl ist;
R₁ Wasserstoff; (C₁-C₆)Alkyl, wahlweise substituiert mit einer oder mehreren primären, sekundären oder tertiären Aminogruppen, Hydroxyoder Carboxygruppen; (C₅-C₆)Cycloalkyl; Aralkyl; Halogen ist;
Y Wasserstoff oder eine -O-R₂-Gruppe ist;
R₂ Wasserstoff; (C₁-C₆)Alkyl, wahlweise substituiert mit einer oder mehreren primären, sekundären oder tertiären Aminogruppen, Hydroxyoder Carboxygruppen; (C₅-C₆)Cycloalkyl; Aralkyl; Aryl; Heteroaryl; Heteroarylalkyl; (C₁-C₆)Acyl ist;
X eine oder mehrere Gruppen ist, die unabhängig voneinander ausgewählt werden aus: Wasserstoff, (C₁-C₆)Alkyl, Hydroxy, (C₁-C₄)Alkoxy, (C₁-C₃)Halogenalkoxy, Amino, (C₁-C₃)Alkylamino, (C₁-C₃)Acylamino, (C₁-C₃)Alkylsulfonylamino, Halogen, Nitro, Cyano, Carboxy, (C₁-C₃)Alkoxycarbonyl, Sulfonyl; (C₁-C₃)Alkylsulfonyl, Aminosulfonyl, (C₁-C₃)Alkylaminosulfonyl, Trifluormethyl;
und worin:
Aralkyl Benzyl oder Phenethyl ist;
Aryl Phenyl, Naphthyl oder Phenyl ist, substituiert mit einem oder zwei Substituenten, ausgewählt aus (C₁-C₆)Alkyl, Hydroxy, (C₁-C₄)Alkoxy, (C₁-C₃)Halogenalkoxy, Amino, (C₁-C₃)Alkylamino, (C₁-C₃)Acylamino, (C₁-C₃)Alkylsulfonylamino, Halogen, Nitro, Cyano, Carboxy, (C₁-C₃)Alkoxycarbonyl, Sulfonyl, (C₁-C₃)Alkylsulfonyl, Aminosulfonyl, (C₁-C₃)Alkylaminosulfonyl, Trifluormethyl;
Heteroaryl Furyl, Thienyl, Imidazolyl, Benzothienyl, Benzofuryl, Pyridyl, Indolyl, Pyrimidyl, Thiazolyl ist;
Heteroarylalkyl Pyridylmethyl, Thienylmethyl, Furylmethyl, Isochinolylmethyl ist.

2. Verbindungen nach Anspruch 1, worin die OR₂-Gruppe an der 8-Position des 1,2-Dihydro-1-oxo-pyrazino-[1,2-a]indol-Heterocyclus-Systems ist.

3. Verbindungen nach Anspruch 1 oder 2, worin R (C₁-C₆)Alkyl ist, R₁ Wasserstoff ist, X Wasserstoff ist und R₂ (C₁-C₆)Alkyl, Aralkyl oder Aryl ist, worin Aralkyl und Aryl wie im Anspruch 1 definiert sind.

4. Pharmazeutische Zusammensetzungen, die als aktiven Bestandteil eine Verbindung nach den Ansprüchen 1 bis 3 in Zusammenmischung mit geeigneten Trägern enthalten.

5. Zusammensetzungen nach Anspruch 4 als Kombinations-Präparate für die sequenzielle oder gleichzeitige Verabreichung, die zusätzlich zur der Verbindung nach Ansprüchen 1 - 3 ein Antitumor-Arzneimittel mit synergistischer Wirkung enthalten.

6. Verwendung der Verbindungen nach den Ansprüchen 1 - 3 für die Herstellung von Antitumor- Arzneimitteln.

## Revendications

1. Composés de formule (I) 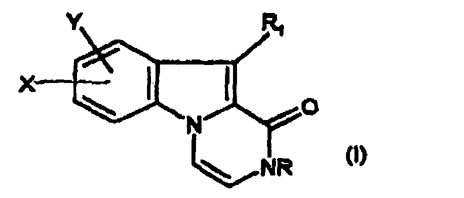 dans laquelle :
R est de l'hydrogène ; un groupe alkyle en C₁ à C₆ facultativement substitué par un ou plusieurs groupes amino primaires, secondaires ou tertiaires, hydroxy ou carboxy ; cycloalkyle en C₅ à C₆ ; aralkyle ; aryle ; hétéroaryle ; hétéroaralkyle ;
R₁ est de l'hydrogène ; un groupe alkyle en C₁ à C₆ facultativement substitué par un ou plusieurs groupes amino primaires, secondaires ou tertiaires, hydroxy ou carboxy ; cycloalkyle en C₅ à C₆ ; aralkyle ; un halogène;
Y est de l'hydrogène ou un groupe -O-R₂ ;
R₂ est de l'hydrogène ; un groupe alkyle en C₁ à C₆ facultativement substitué par un ou plusieurs groupes amino primaires, secondaires ou tertiaires, hydroxy ou carboxy ; cycloalkyle en C₅ à C₆ ; aralkyle ; aryle ; hétéroaryle ; hétéroarylalkyle ; acyle en C₁ à C₆ ;
X est un ou plusieurs groupes indépendamment choisis parmi l'hydrogène, un groupe alkyle en C₁ à C₆, hydroxy, alcoxy en C₁ à C₄, halogénoalcoxy en C₁ à C₃, amino, alkylamino en C₁ à C₃, acylamino en C₁ à C₃, alkylsulfonylamino en C₁ à C₃, halogène, nitro, cyano, carboxy, alcoxycarbonyle en C₁ à C₃, sulfonyle, alkylsulfonyle en C₁ à C₃, aminosulfonyle, alkylaminosulfonyle en C₁ à C₃, trifluorométhyle ;
et dans laquelle
aralkyle est benzyle ou phénéthyle ;
aryle est phényle, naphtyle ou phényle substitué par un ou deux substituants choisis parmi alkyle en C₁ à C₆, hydroxy, alcoxy en C₁ à C₄, halogénoalcoxy en C₁ à C₃, amino, alkylamino en C₁ à C₃, acylamino en C₁ à C₃, alkylsulfonylamino en C₁ à C₃, halogène, nitro, cyano, carboxy, alcoxycarbonyle en C₁ à C₃, sulfonyle, alkylsulfonyle en C₁ à C₃, aminosulfonyle, alkylaminosulfonyle en C₁ à C₃, trifluorométhyle ;
hétéroaryle est furyle, thiényle, imidazolyle, benzothiényle, benzofuryle, pyridyle, indolyle, pyrimidyle, thiazolyle ;
hétéroarylalkyle est pyridylméthyle, thiénylméthyle, furylméthyle, isoquinolylméthyle.

2. Composés selon la revendication 1, dans lesquels le groupe OR₂ est en position 8 du système 1,2-dihydro-1-oxo-pyrazino-[1,2-a]indole hétérocyclique.

3. Composés selon la revendication 1 ou 2, dans lesquels R est un groupe alkyle en C₁ à C₆, R₁ est de l'hydrogène, X est de l'hydrogène et R₂ est un groupe alkyle en C₁ à C₆, aralkyle ou aryle, où aralkyle et aryle sont tels que définis dans la revendication 1.

4. Compositions pharmaceutiques contenant en tant qu'ingrédient actif un composé selon les revendications 1 à 3 mélangé avec un véhicule approprié.

5. Compositions selon la revendication 4, sous la forme de préparations combinées pour l'administration successive ou simultanée, contenant, en plus d'un composé selon les revendications 1 à 3, une substance médicamenteuse antitumorale ayant une action synergique.

6. Utilisation des composés selon les revendications 1 à 3 pour la préparation de médicaments antitumoraux.
